# EUROPEAN PATENT SPECIFICATION

(11) **EP 1 155 381 B1**
(45) Date of publication and mention of the grant of the patent: **30.06.2010**
(21) Application number: 00902395.3
(22) Date of filing: 12.01.2000
(51) Int. Cl.: G06K 9/00, G01N 1/30, G01N 33/48

(54) **MINIMALLY INVASIVE APPARATUS AND METHOD FOR TESTING LESIONS OF THE ORAL CAVITY AND SIMILAR EPITHELIUM**
MINIMAL-INVASIVER APPARAT UND VERFAHREN ZUM TEST ZUM AUFFINDEN VON LÄSIONEN IN DER MUNDHÖHLE UND IN ÄHNLICHEM EPITHEL
APPAREIL ET METHODE PEU EFFRACTIFS DE TEST DE LESIONS DE LA CAVITE ORALE ET DE L'EPITHELIUM SIMILAIRE

(30) Priority: 23.02.1999 US 121255 P; 23.04.1999 US 298219; 23.04.1999 US 298218
(43) Date of publication of application: 21.11.2001
(73) Proprietor: CDX Laboratories, Inc., Suffern, NY 10901 (US)
(72) Inventor: EISEN, Drore, Cincinnati, OH 45237 (US); FRIST, Stephen, Monsey, NY 10952 (US); RECHT, Joel, Monsey, NY 10952 (US)
(74) Representative: Findlay, Alice Rosemary
(86) International application number: PCT/US2000/000743
(87) International publication number: WO 2000/051066

(56) References cited:
- WO-A-00/04833
- US-A- 2 847 990
- US-A- 5 257 182
- US-A- 5 740 270
- SCHULTZ C.P. ET AL.: 'In Situ Infrared Histopathology of Keratinization in Human Oral/Oralpharyngeal Squamous Cell Carcinoma' ONCOLOGY RESEARCH vol. 10, no. 5, May 1998, pages 277 - 286, XP002926859

## Description

### Field of the Invention

The present invention is directed to a minimally invasive apparatus and method for testing lesions of the oral cavity and similar epithelium.

The present invention is directed to determining whether or not there is a likelihood of detection of precancerous or cancerous conditions in epithelial tissue.

While this invention is described for testing lesions of the oral cavity, the methodology related to testing for abnormal keratinization applies to the analysis of any tissue sample in which keratin exists obtained by any medical technique.

### Background of the Invention

Detecting precancerous or cancerous conditions in certain body tissue is important. There are various methodologies of obtaining tissue samples for examination and methodologies for analyzing cellular specimens of the tissue samples.

One area of such activity is in the dental field. Between 5% to 10% of patients in general dental and medical practice have harmless appearing oral lesions which are routinely noticed on oral examination, or which are incidentally observed while performing a cosmetic or other dental procedure. Visual inspection and palpation of these lesions to detect early stage oral cancer is highly unreliable. This is because benign, dysplastic and cancerous lesions are often indistinguishable from each other on clinical inspection. The vast majority of these relatively benign appearing lesions are, in fact, benign. However, at least 6% of these benign appearing lesions may be pre-cancerous or cancerous, and failure to identify these dangerous lesions at an early, treatable stage, is a primary factor in the currently low five-year survival rate for oral cancer.

The dentist or physician who visually detects an oral lesion which is not clearly suggestive of precancer or cancer is faced with a quandary when restricted to the methods and apparatus of the prior art. The only accurate tool currently believed to be available in the prior art to distinguish benign from pre-cancerous and cancerous oral lesions is a lacerational or scalpel biopsy of the lesion followed by histological examination of the excised tissue. In a scalpel biopsy, a variety of surgical cutting instruments are used to obtain a tissue sample. If such a scalpel biopsy removes a part of the lesion it is referred to as an "incisional" biopsy, while if it removes the entire lesion it is referred to as an "excisional" biopsy.

In either case, a scalpel biopsy is a painful, lacerational, highly invasive procedure. Typical instruments for this purpose include, but are not limited to a flat scalpel blade, a round scalpel blade (punch biopsy) and scissors. Local anesthesia is always required. Considerable bleeding from the wound is common and suturing is often necessary. For these reasons, primary care dentists and physicians, those clinicians who most often encounter benign appearing oral lesions, are reluctant to perform a scalpel biopsy. When necessary, these clinicians will therefore generally refer the patient to an oral surgeon or oral pathologist for the procedure. Since as many as 5% to 10% of all patients in a typical dental or general adult medical practice may have such visible oral lesions, many of which are likely to be benign, performing a scalpel oral biopsy in the primary care setting or referral to a specialist for such performance is reserved for only the most clinically suggestive lesions. Yet, as has repeatedly been shown, pre-cancerous and cancerous oral lesions often mimic benign lesions. Lacking the subject invention, these pre-cancerous or cancerous, but benign appearing, oral lesions typically do not receive any immediate diagnostic evaluation and are thus allowed to progress to an advanced stage of oral cancer. Once such progression is underway and continues untreated, the patient's chances for recovery diminishes.

A prior art approach which has attempted to address this problem in testing lesions of the oral cavity was the use of cytology. In this approach, a sample of cells which was naturally exfoliated from the surface of a lesion into mucous or saliva is examined microscopically. While cytology is commonly used to detect precancer and cancer in other body sites, it has not proven to be useful in the oral cavity because of its low sensitivity, i.e. its high false negative rate. It is believed that this high false negative rate is in part due to the fact that many oral lesions have an overlying keratin layer which limits availability to the lesion surface of naturally exfoliated abnormal cells. In one large study, oral cytology was found to have a false negative rate of 30%. This means that 30% of oral lesions determined to, in fact, be precancerous or cancerous on scalpel biopsy and histology were falsely reported as "negative" using oral cytology. Due to its unreliable sensitivity, prior art cytologic technique is rarely used to test oral lesions or similar keratinized epithelial lesions for precancer or cancer.

'In Situ Infrared Histopathology of Keritinization in Human Oral/Oralpharyneal Squamous Cell Carcinoma' ONCOLOGY RESEARCH vol. 10, no. 5, May 1998, pages 277 - 286, XP002926859 discloses a method of producing biochemical images of tissue based on infrared spectra of distributions of various biochemical markers, such as keratin and DNA.

### Summary of the invention

In accordance with the invention as defined in claims 1 and 28, a unique processing system to analyse cellular specimens for abnormal keratinisation is described. A cytological or cellular sample of an oral lesion is taken from a patient for analysis. This sample is obtained by means of a non-scalpel instrument which is sufficiently abrasive to penetrate all three layers (basal, intermediate, and superficial) of the oral epithelium. In the preferred embodiment, this trans-epithelial sample is obtained by means of pressing and rotating a circular stiff nylon brush several times over the entire lesion surface.

As an important feature of the subject invention, a cellular sample is examined with the aid of an image recognition system designed to identify minimal evidence of pre-cancerous and cancerous change. In accordance with the invention, the system can detect small numbers of abnormal cells distributed among the large number of normal cells obtained during the sampling procedure.

In this alternate or additional embodiment, the subject invention preferably overcomes the limitations and difficulties associated with analysis of cellular specimens for abnormal characteristics by providing an image recognition system which detects characteristics relating to abnormal keratinization of the cells. Preferably, these characteristics include color saturation associated with such abnormal keratinization.

In the preferred embodiment illustrating the invention, the system described overcomes the sensitivity limitations of prior art oral cytologic technique by combining innovations in oral pathology, namely: 1) a non-scalpel cellular sample of all three layers of the oral epithelium; and 2) subjecting this novel sample to inspection by the novel image recognition system specifically designed to detect minimal evidence of early precancerous change in a trans-epithelial sample from an oral lesion, or other lesion with similar epithelia. This novel image recognition system which is the subject of this patent application preferably analyzes for the presence of abnormal keratinization, by detecting predetermined characteristics of color saturation.

For purposes of this patent application, the prior art scalpel procedure is defined as lacerational, whereas the sample collection device herein is non-lacerational and therefore minimally invasive. To the extent that an abrasive brush has characteristics that may cause minor discomfort and/or bleeding, there is substantial difference between the prior art scalpel trauma and the minimal trauma associated with the present procedure.

Thus, in the preferred embodiment of the invention, the image processing system combines: 1) sensitivity to the presence of abnormal cellular morphology obtained from any or all of the three layers of the novel trans-epithelial cellular sample with 2) sensitivity to the presence of abnormally keratinized cells as are commonly found in any or all of the three layers of the epithelia and also obtained by means of the novel trans-epithelial cellular sample of the subject invention. Thus, the keratin component, which presented an obstacle to prior art oral cytology, is both penetrated, to ensure that any underlying abnormal basal cell morphology is available for analysis, and productively utilized, as a means of increasing the method's overall sensitivity to evidence of precancerous and cancerous change.

In a preferred embodiment, the image recognition system selects the most suspect abnormal cells and cell clusters among the sample, and displays these cells and cell clusters on a video monitor for expert review.

In the preferred embodiment, the image recognition system also provides a color printout of those suspect cells and cell clusters selected by the expert reviewer as representative of the case.

In the preferred embodiment, the image recognition system selects abnormal cells based on morphological characteristics and on overall resemblance to abnormal cells on which it has been trained.

In the preferred embodiment of the invention, the image recognition system is directed to detection of abnormality of the oral cavity by including a function to detect abnormal keratin as is often found in dysplastic and cancerous oral tissue.

In the preferred embodiment of the invention, the image recognition system is directed to detection of abnormal keratin by being programmed to detect a threshold level of color saturation associated with "hyalinization" or the stained appearance of such abnormal keratin.

In a further preferred embodiment of the invention, the image recognition is directed to the combination of morphological cellular change associated with pre-cancer and cancer and the appearance of abnormal keratin as produced by pre-cancerous and cancerous cells of the oral cavity and similar epithelia.

In one embodiment of the invention, the image recognition system may be constructed through modification of image recognition systems currently manufactured and sold to detect abnormal cells spontaneously exfoliated from non-keratinized lesions such as cervical lesions.

### Advantages of the Invention over the Prior Art:

The invention consists of a method and system which for the first time provides accurate evaluation of oral lesions without requiring the performance of a scalpel biopsy. A primary objective of the subject invention is to accurately distinguish pre-cancerous and cancerous oral lesions from benign oral lesions without the pain, bleeding, and tissue wound that typically result from the prior art technique of scalpel biopsy and histology.

In a multi-center clinical trial with over 800 patients performed at 35 U.S. Academic Dental Centers, the sensitivity and specificity of the subject invention was compared to the prior art technique of scalpel biopsy and histology. In this double blind study, the subject invention was found to detect 100% of the pre-cancerous and cancerous lesions detected by the prior art technique. The subject invention thus had a 0% false negative rate in this study. As noted above, this contrasts with false negative rates as high as 30% commonly associated with prior art oral cytology. In addition, the subject invention had less than a 1 % false positive rate in this study. In this study, the subject invention also detected pre-cancer or cancer in approximately 15 patients whose lesions did not visually appear suspicious enough to expert examiners to warrant a scalpel biopsy. These outcomes represent lives that were potentially saved by the subject invention.

An additional advantage of the subject invention over the prior art diagnostic technique is greater sensitivity to the detection of pre-cancer and cancer in large multi-focal oral lesions. This is because of the larger sampling area obtained by the brush biopsy technique of the subject invention when compared to the smaller area sampled by a traditional incisional scalpel biopsy.

An additional advantage of the subject invention is that patients which have chronic oral lesions can have these lesions followed over time by repeated testing using a minimally invasive procedure.

An additional advantage is that it allows accurate, minimally invasive testing of lesions from areas of the body outside of the oral cavity where a keratin layer that limits the accuracy of prior art cytological technique may be present. Several such areas are the larynx, pharynx, esophagus, and vulva.

An additional advantage of the subject invention is that it allows diagnosis of non-cancerous conditions, such as candidiasis, herpes, geographic tongue, lichenplanus, human papilloma virus, and others.

### Brief Description of the Drawing

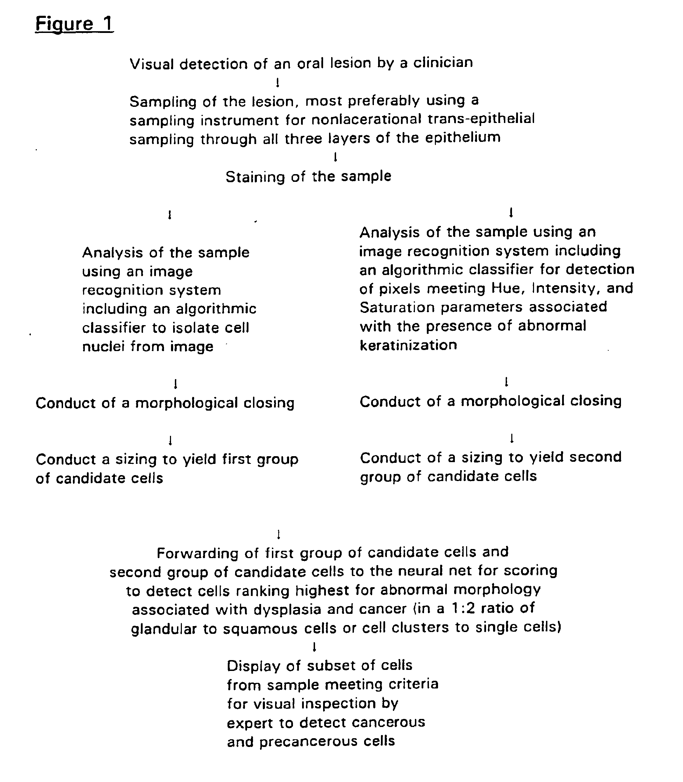

Figure 1 is a flowchart illustrating the method of one embodiment.
Figure 2 is a flowchart illustrating the method of an alternate embodiment.
Figure 3 is a flowchart illustrating the method of a third embodiment.
Figure 4 is a cross sectional view of an section of epithelium including precancerous or cancerous cells, showing the extent of sampling obtained using exfoliative cytology.

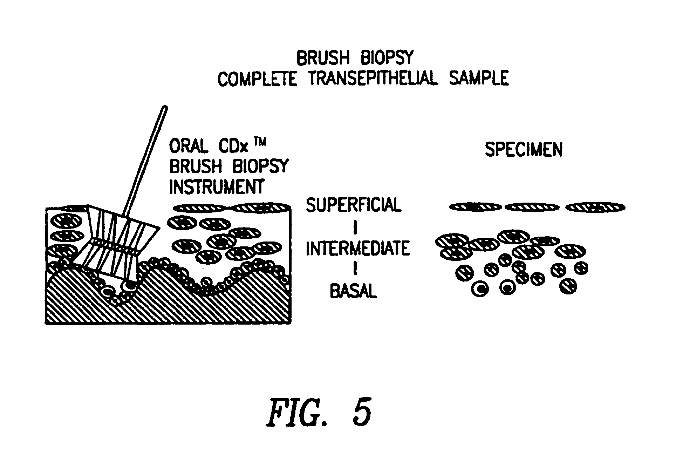

Figure 5 is a cross sectional view of an section of epithelium including precancerous or cancerous cells, showing the extent of sampling obtained using the brush biopsy technique.

### Detailed Description of the invention and the Preferred Embodiments

It is known that the functional differentiation of euplastic tissues to form keratinized stratified squamous epithelium is characterized by certain important morphological features. Frost, for example, discusses this differentiation, and the prototypical morphology at length. See, John K. Frost, The Cell in Health and Disease: An Evaluation of Cellular Morphologic Expression of Biologic Behavior, 2nd edition, Chapter 11, in Monographs in Clinical Cytology, vol. 2 (New York: Karger 1986). Specifically, such cells display a central nucleus, a thread-like chromatin pattern, karyopyknosis with maturation, intercellular bridges, stratification, keratinization, thinning, orientation parallel to the basement membrane and lumen, and exfoliation as single cells.

In accordance with the present invention, an automated, computer implemented, system is provided for detection of such differentiation, particularly for cells of the oral cavity and similar epithelium. As such, the invention provides a means for alerting physicians to the presence of cancerous or precancerous cells at an early stage by overcoming the disadvantages of the prior art which has tended to avoid early detection.

A sample of cells from the oral cavity or similar epithelia is processed by an image analysis system for detection of characteristics associated with dysplasia or cancer. The sample is a sample taken using a nonlacerational sampling device. The sample is sent to an automated image processing system for detection of abnormal keratinization.

It is important that a transepithelial sample be taken from the oral cavity or similar epithelia, the sample being obtained using a nonlacerational sampling device. This cytological or cellular sample of the entire epithelial thickness of an oral lesion is obtained using a non-scalpel instrument which is sufficiently abrasive to penetrate all three layers (basal, intermediate, and superficial) of the oral epithelium. In the preferred embodiment, the device is the sampling instrument disclosed in U.S. Patent No. 6 258 044, or is the Spirabrush^{™}, available from The Trylon Corporation of Torrance, California, or the like. In the preferred embodiment, this trans-epithelial sample is obtained by means of pressing and rotating a circular stiff nylon brush several times over the entire lesion surface. This sample is then analyzed by an automated image processing system for detection of morphology or characteristics typical of dysplasia or cancer.

The transepithelial sample is analyzed by an image processing system for detection of the presence of abnormal keratinization. In a further preferred embodiment, the sample is also analyzed for the presence of other characteristics or morphology associated with dysplasia or cancer. If desired, this analysis can be done with any cellular sample, whether a sample obtained by histology or cytology, and can include cells naturally sloughed off, cells intentionally rubbed off, or a tissue sample from an oral biopsy. Although such techniques can be used in conjunction with the invention, however, they do not constitute the preferred embodiment, as it is preferred that a brush biopsy be performed.

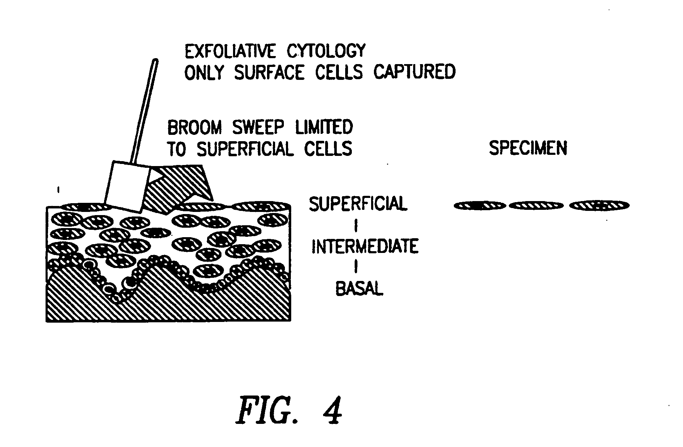

As shown in Figure 4, in the prior art method of exfoliative cytology a non-abrasive sweep is conducted of the epithelial surface in a region of interest which typically only captures surface and exfoliated cells from the epithelial area. Abnormal cells located below the surface, for example, cells located at the basal layer, will therefore often be missed using this superficial sampling. As a result, the specimen sent for analysis, will lack these deeper cells, and may therefore result in a false negative diagnosis. In accordance with the preferred embodiment of the present invention, in contrast, a brush biopsy is taken, using the brush biopsy instrument disclosed, the brush being used to penetrate below the surface of the epithelium and obtain cells from all three epithelial layers. Thus, the specimen sent for testing includes abnormal cells regardless of whether they are cells from the superficial layer or the deeper intermediate and basal layers, providing a more complete and accurate cellular sample for analysis.

In the preferred embodiment, the subject invention thus combines two innovations in oral pathology, namely, a cellular sample of all three layers of the oral epithelium obtained without the use of a scalpel or similar lacerational instrument, with an analysis of that sample by a novel image recognition system specifically designed to detect minimal evidence of early precancerous change in that trans-epithelial sample from an oral lesion, or other lesion with similar epithelia. In the preferred embodiment of the invention, the image processing system combines sensitivity to the presence of abnormal cellular morphology obtained from any of the three layers of the epithelia with sensitivity to the presence of abnormally keratinized cells as are found in any of the layers of the epithelia. In contrast to the prior art, the keratin component, which previously presented an obstacle to prior art oral cytology, is penetrated to ensure that any underlying abnormal intermediate and basal cell morphology is available for analysis and productively utilized as a means of increasing the method's overall sensitivity to evidence of precancerous and cancerous change.

In one preferred embodiment, the invention utilizes a modified version of the commercially available PAPNET system, currently sold by Neuromedical Systems, Inc. of Upper Saddle River, New Jersey and Suffern, New York. Further details of such systems are described in U.S. Patent No. 4,965,725, entitled "Neural Network Based Automated Cytological Specimen Classification System and Method"; U.S. Patent No. 5,257,182, entitled "Morphological Classification System and Method"; U.S. Patent No. 5,287,272, entitled "Automated Cytological Specimen Classification System and Method"; U.S. Patent No. 5,232,207, entitled "Inspection Apparatus and Method with Inspection Auditing for images Presented on a Display"; U.S. Patent No. 5,544,650, entitled "Automated Specimen Classification System and Method"; U.S. Patent No. 5,625,705, entitled "Intensity Texture Based Classification System and Method"; U.S. Patent No. 5,629,766, entitled "Global MTF Measurement System"; U.S. Patent No. 5,655,029, entitled "Device and Method for Facilitating Inspection of a Specimen"; U.S. Patent No. 5,659,421, entitled "Slide Positioning and Holding Device"; and U.S. Patent No. 5,740,270. entitled "Automated Cytological Specimen Classification System and Method". In alternative embodiments, the invention utilizes a modified computer image recognition system having similar or equivalent capability to detect and present abnormal cells within certain predetermined parameters.

Currently, oral lesions which are routinely noticed on oral examination, or are incidentally observed while performing a cosmetic or other dental procedure, are only rarely biopsied or tested to detect early stage cancer. The dentist or physician, or other expert who visually detects such lesions can now routinely test them to detect abnormality with a minimum of discomfort to the patient.

Such testing is conducted to obtain a transepithelial sample of the lesion which can them be sent for staining and subsequent analysis by a computer implemented system. Accordingly, in the first step, a transepithelial sample is taken from the patient's oral lesion.

The cytological or cellular sample is taken of the entire epithelial thickness of an oral lesion by means of a nonlacerational or nonscalpel instrument which is sufficiently abrasive to penetrate all three layers (basal, intermediate, and superficial) of the oral epithelium. Preferably, this trans- epithelial sample is obtained by means of pressing and rotating a circular stiff nylon brush several times over the entire lesion surface. In a preferred embodiment, the sample is taken using the brush disclosed in U.S. Patent No. 6 258 044 or the Spirabrush^{™}, available from The Trylon Corporation of Torrance, California.

Upon sampling of the lesion, the sample is stained preferably using the modified Papanicolaou stain, as is well known in the art. This stained sample is then imaged and analyzed using an image recognition system which selects abnormal cells based on a combination of abnormal morphology and abnormal keratinization particularly the characteristics associated with lesions of the oral cavity and similar epithelia.

In the preferred embodiment of the invention, the image recognition system first processes the image through an algorithmic classifier and then sends the processed data to a neural net work. The algorithmic classifier locates a first group of candidate objects within the image which could be the nuclei of cells. In accordance with the invention, the algorithmic classifier has been modified to also locate a second group of candidate objects within the image, these being cells displaying the abnormal keratinization typical of precancerous and cancerous lesions of the oral cavity. The neural net then scores cells for the presence of other morphological features associated with cancerous cells.

In one preferred embodiment of the invention, these two candidate groups are both sent to the neural net for scoring, and the highest ranking objects from the combined two groups are displayed to the expert reviewer. In an alternate embodiment of the invention, those cells displaying abnormal keratinization do not compete with other cells in the neural net stage of analysis; rather, all abnormally keratinized cells are forwarded for display to the expert reviewer.

In one preferred embodiment, the stained sample is analyzed by a modified PAPNET system, the system being modified as described herein. Such PAPNET systems are commercially available image recognition systems which select the most suspect abnormal cells and cell clusters among those presented in a stained sample, and then display these cells and cell clusters on a video monitor for expert review.

In the present state of such PAPNET systems, the programming of the system is set to detect and display the top 64 glandular cells or top 64 clusters of cells, and the top 64 squamous cells or top 64 single cells, the ratio of glandular to squamous cells or cell clusters to single cells being in a 1:1 ratio, these top cells being those cells ranked highest for abnormalities by the system.

In accordance with the present invention, the programming of the system is modified to vary this detection ratio to enhance the system's performance on oral brush biopsies of the oral mucosa. In accordance with the invention, the system is reprogrammed to detect and display the top 64 clusters of cells and the top 128 single cells, in a 1 :2 ratio. Although a 1 :2 ratio is used in the preferred embodiment, in other embodiments modifications can be made to the controller such that a 1 :3 ratio, or some other ratio higher than 1:1 can be employed. Accordingly, by modification of this detection parameter the detection capabilities for abnormalities in oral lesions is improved.

In its current form, the commercially available PAPNET system is programmed to analyze the visual image of the stained sample to detect abnormalities consistent with the presence of a cancerous or precancerous condition. In accordance with the present invention, the current programming is still utilized and such abnormalities are located. However, in addition thereto, the system is reprogrammed such that a further set of parameters are introduced to enhance the system's detection capabilities, by isolating a second group of candidate cells. Specifically, in the preferred embodiment of the invention, the image recognition system is directed to enhance its detection of abnormalities of the oral cavity by including a function to detect abnormalities such as are characteristic of dysplastic and cancerous oral tissue. In the preferred embodiment, the system detects abnormal keratin. These keratinized cells are then either forwarded to the neural net for detection of abnormalities consistent with cancer or precancer, or are forwarded directly to an expert for analysis.

Another method to detect abnormal samples is to set threshold levels for morphology and/or color, which threshold levels indicate, if exceeded, that there is a likelihood of abnormal cells in the sample being analyzed.

Research has indicated that lesions of the oral cavity and similar epithelium often display a progressive keratinization. This process of keratinization occurs throughout the epithelial layers. These keratins typically occur uniformly throughout the cytoplasm. In accordance with the present invention, several morphologic features present in the routine Papanicolaou stain are used to facilitate the detection and identification of these keratins. The most distinctive characteristic of the presence of the more mature keratins is a hyalinization, or glassy appearance to the cytoplasm. As Frost has described, for example, this hyaline character often imparts to the cytoplasm "the impression of viewing a brilliantly gleaming colored glass of a stained glass window, with the sun beaming in from behind." Other observers have described the appearance as "shiny", "metallic" or "glowing." A second important characteristic associated with these keratins is the presence of a particular color. As the process of keratinization develops, samples obtained by staining change from basophilia (i.e. green to blue) toward acidophilia (e.g. yellow or orange to red). In accordance with the present invention, an orange to red color is looked for, or a deep 'Halloween orange'.

Accordingly, in the preferred embodiment of the invention, the PAPNET system is redesigned or reprogrammed to detect "glowing" or glassy, orange - red cytoplasms, as are associated with abnormal keratinization of oral and similar epithelia. In the preferred embodiment, the algorithmic classifier is programmed to take the cells of the stained sample and to further perform an analysis of their color. In one embodiment, each pixel of the image is separately screened or tested for distinctive color characteristics. In an alternative embodiment, the cells are first screened and detected for abnormality by the system using the standard parameters of the PAPNET system (with a modified detection ratio as described above) and are then analyzed for distinctive color.

In this color detection step of the preferred embodiment, every pixel of the image is preferably measured to detect the pixel's Hue (H), Intensity (I) and Saturation (S). The target Hue sought is an orange - red, such as that commonly associated with abnormally keratinized cytoplasm. In one preferred embodiment, the system searches for a Hue of approximately 46-82 on a scale ranging from 0-255. In addition to Hue, the target pixels which the system searches for are those cells which further have a high Intensity and a high Saturation. Specifically, in one preferred embodiment, the system is set to search for and detect pixels with an Intensity of 100-255 on a range of 0-255. Likewise, in the preferred embodiment, the system is set to detect a Saturation of 43-255, also on a range of 0-255.

Having been set with the desired parameters of H, I and S, in the preferred embodiment the system is programmed to analyze each pixel of the sample image and to narrow down the target pixels of interest to those pixels displaying all of the preferred parameters of desired Hue, Intensity and Saturation. RGB analysis could also be employed.

Once the pixels with the desired color characteristics have been isolated, a morphological closing, i.e. a dilation and an erosion, is mathematically performed by the system to find round objects. Such closings are known in the art, and are used, for example, elsewhere in the prior unmodified PAPNET system for purposes unrelated to the detection of abnormal keratin. Likewise, Pratt describes such closings and the mathematics of the same. See, William K. Pratt, Digital Image Processing, Second Edition, Chapter 15, Morphological Image Processing (New York: John Wiley & Sons).

In accordance with the present invention, the morphological closing operation on these pixels with the desired color characteristics is performed using a 9x9 (octogonal) structuring element. (The 14x 14 structuring element used by the PAPNET system in a separate step unrelated to color processing can still be used, however, in that separate step of the image processing). The 9x9 structuring element used in this stage of the process tends to find round objects, and will find those which are approximately 2 - 18 microns in diameter.

Once this closing has been performed, a sizing is performed. In this step, objects under 10 microns in diameter are rejected from consideration. Such filtration of objects based on size is well known in the art, having been used in other applications, among them the unmodified PAPNET system, and is discussed, for example, in U.S. Patent No. 5,257,182 issued to Luck et al., and entitled "Morphological Classification System and Method".

Once this sizing step has been conducted, the system has now effectively selected round objects with a glowing cytoplasm that meet the criteria of abnormally keratinized cells of the oral cavity. The system then cuts out a window of approximately 48 microns by 48 microns around the centroid of each such object, forming the second group of candidate objects.

In the preferred embodiment of the invention, the first group of candidate objects (those objects which have been isolated by the algorithmic classifier without reference to color characteristics) and the second group of candidate objects (those cells isolated based on color characteristics that indicate the presence of abnormal keratinization) are both sent to the neural net for scoring, and the highest ranking objects from the combined two groups are displayed to the expert reviewer.

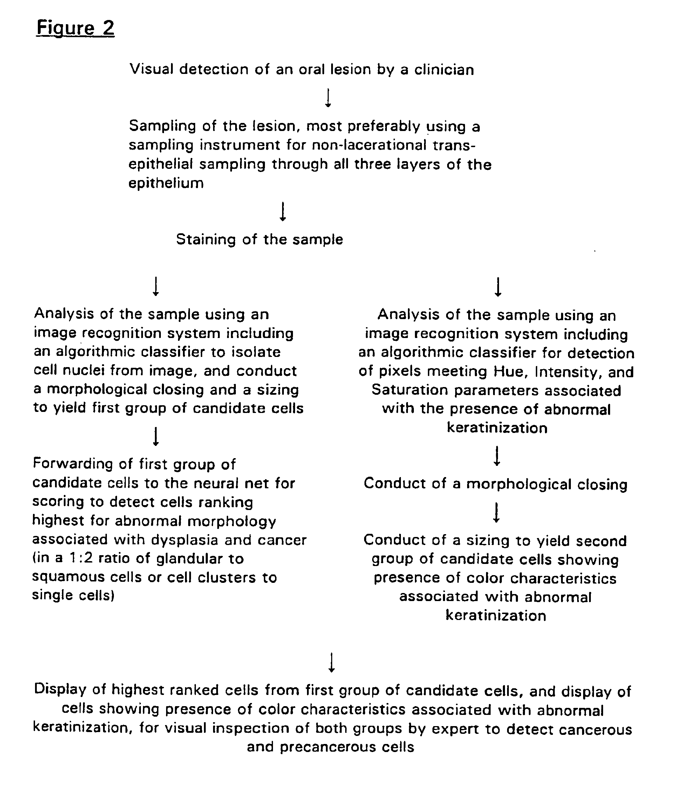

In an alternate embodiment of the invention, as shown in Figure 2, those cells displaying abnormal keratinization do not compete with other cells in the neural net stage of analysis. Rather, the first group of candidate cells is scored by the neural net, and the second group of candidate cells bypasses the neural net and is directly forwarded for display to the expert reviewer.

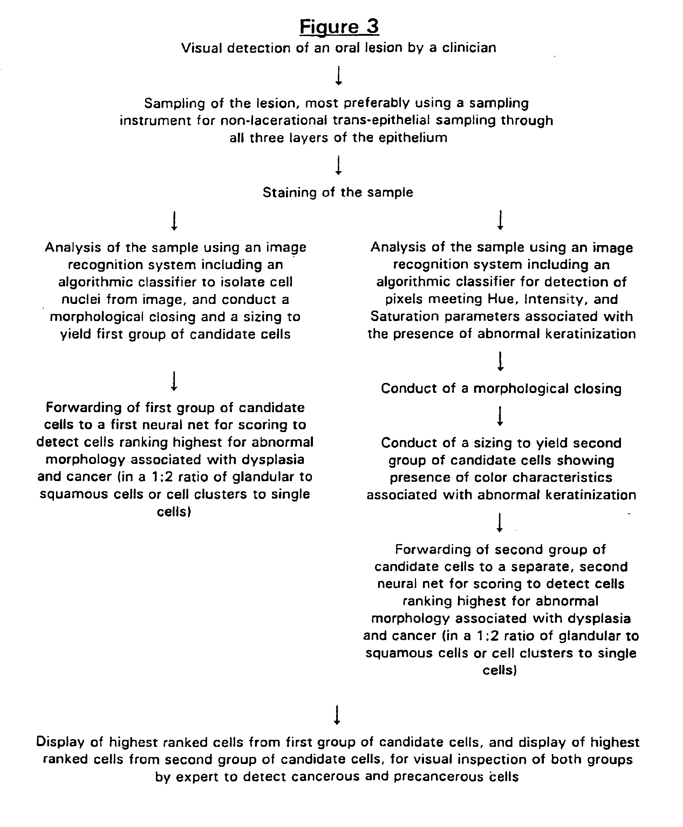

In yet a further alternate embodiment of the invention, as shown in Figure 3, those cells displaying abnormal keratinization are sent to a separate, second neural net for independent ranking by a neural net, but also without having to compete with other cells in the neural net stage of analysis. Thus, the first group of candidate cells is scored by a first neural net, and the second group of candidate cells is scored by a second neural net, with each group of cells being forwarded for display to the expert reviewer.

A pathologist, cytologist or other expert reviewer can then visually examine the objects forwarded for the display, the objects being displayed on a video monitor. Upon visual inspection, the reviewer can then make a determination whether a cancerous or precancerous condition is present. Preferably, in accordance with the invention, the image recognition system also provides a color printout of those suspect cells and cell clusters selected by the expert reviewer as representative of the case.

An alternative embodiment to scoring and displaying the cells to an expert reviewer is to program the system to set certain threshold levels both for morphological abnormalities and/or abnormal keratinization. When those threshold levels are exceeded, the system will automatically produce a visualization of only such specific cells and not those highest scored or highest ranked on the specimen regardless of whether or not the scores have exceeded the threshold level.

In a further embodiment of the invention, the system can be programmed to detect and display basal cells using a basal cell detector in the algorithmic classifier. Alternatively, the pathologist can merely examine the cells in the sample with a standard microscope to detect the presence of basal cells. This embodiment provides a "fail-safe feature" within the overall testing process to ensure that a transepithelial sample has been obtained, i.e. that the cellular sample includes cells from all three layers of the epithelia. Should the pathologist determine that basal cells are not, in fact, present, the incomplete sample can be disregarded if negative and sampling can be repeated to attempt to obtain a transepithelial sample for analysis. In this manner, the pathologist will not rely on the incomplete sample for the test results. Thus, if the initial sampling fails to obtain a transepithelial sample, that deficiency is detected in advance and the patient will not obtain a false sense of security from a false negative.

The PAPNET system, as is commercially available was designed for a platform with a neural network which has relatively slow processing speeds. Because of the large number of cells in the original sample requiring examination, and the speed of the original PAPNET neural network, a multiple stage analysis was performed in which the population of cells to be analyzed by the neural network was necessarily reduced at each stage to allow the overall process to run in a reasonable time. For example, if 500,000 cells were in a sample, a first analytical stage applying algorithmic, first stage neural network or other analytical techniques could reduce that population to approximately 50,000 cells, which could then be realistically examined by the processing speed of the then available neural networks.

Due to the increased speed of microprocessors and computers, the decisional analysis to determine either threshold level crossings, color analysis or morphological analysis can be accomplished with a single stage neural network. A single stage neural network is meant to indicate that there may be different layers within the neural network, but the entirely of the process can occur in a single stage without prior elimination of objects requiring neural network analysis.

With current processors, such as a Pentium 300, a single neural network stage can be implemented in which the entirety of the population of sample cells can be analyzed in a sufficiently short period of time to realize practical operation.

Having described this invention with regard to specific embodiments, it is to be understood that the description is not meant as a limitation since further embodiments, modifications and variations may be apparent or may suggest themselves to those skilled in the art. It is intended that the present application cover all such embodiments, modifications and variations.

## Claims

1. Apparatus to detect abnormal cells in epithelial tissue of the body comprising:
transepithelial non-lacterational sampling means to collect cells from at least two layers of said epithelial tissue,
analytical apparatus comprising an image recognition system to detect and display selected cells of said cells and to analyze said selected cells collected by said transepithelial sampling means to detect abnormally keritinized cells or cells having morphological cellular change associated with pre-cancer and cancer,
wherein said trans-epithelial non-lacerational sampling means is sufficiently abrasive to collect cells from three layers of said epithelial tissue, said three layers comprising superficial, intermediate and basal layers,
wherein said image recognition system is adapted to detect whether or not cells from said basal layer are detected.

2. Apparatus to detect abnormal cells in epithelial tissue of the body according to claim 1, wherein said epithelial tissue comprises keratinized cells.

3. Apparatus to detect abnormal cells in epithelial tissue of the body according to claim 1 or 2, wherein said analytical apparatus is adapted to terminate said analysis if cells from said basal layers are not detected.

4. Apparatus to detect abnormal cells in epithelial tissue of the body according to any of the preceding claims, wherein said transepithelial sampling means comprises an abrasive sampling structure.

5. Apparatus to detect abnormal cells in epithelial tissue of the body according to claim 4, wherein said sampling structure comprises a brush.

6. Apparatus to detect abnormal cells in epithelial tissue of the body according to claim 5, wherein said brush comprises bristle of sufficient stiffness to reach said basal layer.

7. Apparatus to detect abnormal cells in epithelian tissue of the body according to claim 6, wherein said brush comprises a circular stiff nylon brush.

8. Apparatus to detect cells with abnormal morphology in epithelian tissue of the body according to claims 1 - 3 or 6, wherein said epithelial tissue is located in the areas of the body selected among the group comprising the larynx, pharynx, esophagus and vulva.

9. Apparatus to detect anormal cells in epithelial tissue of the body according to claims 1 - 3, the image recognition system adapted to image said abnormal cells.

10. Apparatus to detect abnormal cells in epithelial tissue of the body according to claim 9, wherein said image recognition system comprises means to select the most suspect abnormal cells among the cells sampled and means to image said suspect cells.

11. Apparatus to detect abnormal cells in epithelial tissue of the body according to claim 10, wherein said image recognition system further comprises means to produce a color image of said suspect cells.

12. Apparatus to detect abnormal cells in epithelial tissue of the body according to claims 9 - 11, wherein said image recognition system is adapted to display said abnormal cells to be viewed.

13. Apparatus to detect abnormal cells in epithelial tissue of the body according to claims 9 - 12, wherein said image recognition system is adapted to detect cells collected by said sampling means which exhibit abnormal morphology, said image recognition system further comprises an algorithmic classifier.

14. Apparatus to detect abnormal cells in epithelial tissue of the body according to claims 9 - 13, wherein said apparatus further comprises means to conduct a morphological closing.

15. Apparatus to detect abnormal cells in epithelial tissue of the body according to claim 13, wherein said algorithmic classifier selects a first group of candidate cells, said apparatus further comprising means to conduct a sizing on said first group of candidate cells.

16. Apparatus to detect abnormal cells in epithelial tissue of the body according to claims 9 - 15, wherein said apparatus further comprises means to produce a second group of candidate cells related to the detected color properties.

17. Apparatus to detect abnormal cells in epithelial tissue of the body according to claim 16 as dependent on claim 15, further comprising means to analyse said first and second groups of candidate cells to rank those cells having the highest probability for being abnormal cells.

18. Apparatus to detect abnormal cells in epithelial tissue of the body according to claim 17, further comprising means to display a selected group of said cells having said highest probability.

19. Apparatus to detect abnormal cells in epithelial tissue of the body according to claims 15 - 18, wherein said apparatus comprises means to detect cell size of approximately 2-18 µm in diameter.

20. Apparatus to detect abnormal cells in epithelial tissue of the body according to claims 15 - 18, wherein said apparatus further comprises means to reject objects under 10 µm in diameter.

21. Apparatus to detect abnormal cells in epithelial tissue of the body according to claim 9, wherein said apparatus which analyses said stained cells includes an algorithmic classifier to isolate cell nuclei from the image produced by said image recognition system, further comprising means to conduct a morphological closing and to conduct a sizing to yield a first group of candidate cells.

22. Apparatus to detect abnormal cells in epithelial tissue of the body according to claim 9, wherein said image recognition system includes an algorithmic classifier for detecting parameters associated with the presence of keratin, conducting a further morphological closing of said sample analysing the presence of keratin, and conducting a sizing to yield a second group of candidate cells from said cells which contain keratin and have had a morphological closing.

23. Apparatus to detect abnormal cells in epithelial tissue of the body according to claim 16, further comprising a neural network to analyse said first group of candidate cells and said second group of candidate cells to rank those having the highest abnormal morphology, and means to display a subset of said cells.

24. Apparatus to detect abnormal cells in epithelial tissue of the body according to claim 23, wherein said image recognition system comprises means to display the highest ranked cells from the first group of candidate cells and display cells showing presence of color characteristics associated with abnormal keritinization for visual inspection.

25. Apparatus to detect abnormal cells in epithelial tissue of the body according to claim 23 or 24, wherein said image recognition system and said analytical apparatus comprises means to set a threshold level relating to the characteristics of the cells sampled by said sampling means, and means to detect when the characteristics of said cells of said sampling means exceed said threshold level.

26. Apparatus to detect abnormal cells in epithelial tissue of the body according to claim 25, wherein the characteristics of the cells sampled for which a threshold level is set relate to the color characteristics thereof.

27. Apparatus to detect abnormal cells in epithelial tissue of the body according to claim 26, wherein the color characteristics relate to the appearance of abnormal keritinization.

28. A method to detect abnormal cells in sample cells of epithelial tissue of the body, such sample cells having been collected from non-lacerational means sufficiently abrasive to collect said sample cells from among the superficial, intermediate and basal layers of said epithelial tissue, wherein said method analyses the sample cells, said method comprising:
using an image recognition system to detect and display selected cells of said sample cells and to analyse the selected cells to detect abnormally keritinized cell or cells having morphological cellular change associated with pre-cancer and cancer, wherein said method further includes the step of analysing the sample cells to determine if sample cells have been collected from the basal layer.

29. A method to detect abnormal cells in sample cells of epithelial tissue of the body according to claim 28, wherein said epithelial tissue is oral mucosa.

30. A method to detect abnormal cells in sample cells of epithelial tissue of the body according to either claim 28 or 29, further comprising the step of imaging said abnormally keritinized cells.

31. A method to detect abnormal cells in sample cells of epithelial tissue of the body according to claims 28 - 30, further comprising the step of selecting the most suspect abnormal cells among the sample cells.

32. A method to detect abnormal cells in sample cells of epithelial tissue of the body according to claims 28 - 31, further comprising the step of detecting color properties associated with abnormal keritinization.

33. A method to detect abnormal cells in sample cells of epithelial tissue of the body according to claims 28 - 32, further comprising the steps of detecting which of said sample cells exhibit abnormal morphology and scoring said cells which exhibit abnormal morphology.

34. A method to detect abnormal cells in sample cells of epithelial tissue of the body according to claim 32, further comprising the step of conducting a morphological closing.

35. A method to detect abnormal cells in sample cells of epithelial tissue of the body according to claim 34, further comprising conducting a sizing to yield a first group of candidate cells.

36. A method to detect abnormal cells in sample cells of epithelial tissue of the body according to claim 35, further comprising producing a second group of candidate cells related to color saturation characteristics.

37. A method to detect abnormal cells in sample cells of epithelial tissue of the body according to claim 36, further comprising the step of analysing said first and second groups of candidate cells to rank those cells having the highest probability for being abnormal cells.

38. A method to detect abnormal cells in sample cells of epithelial tissue of the body according to claims 28 - 37, comprising the step of staining said cells to produce a stained appearance of abnormal keratin.

39. A method to detect abnormal cells in sample cells of epithelial tissue of the body according to claim 38, comprising the step of algorithmically classifying said stained cells, imaging said stained cells, isolating cell nuclei from said image, conducting a morphological closing and conducting a sizing to yield a first group of candidate cells.

40. A method to detect abnormal cells in sample cells of epithelial tissue of the body according to claim 39, further comprising the steps of conducting a morphological closing of cells associated with the presence of abnormal keritinization and conducting a sizing of said cells associated with the presence of abnormal keritinization to yield a second group of candidate cells.

41. A method to detect abnormal cells in sample cells of epithelial tissue of the body according to either claim 36 or claim 37, further comprising the step of analysing said first group of candidate cells and said second group of candidate cells to rank those having the highest abnormal morphology and displaying a subset of said cells.

42. A method to detect abnormal cells in sample cells of epithelial tissue of the body according to claim 41, further comprising the step of displaying the highest ranked cells from the second group of candidate cells which show the presence of color characteristics associated with abnormal keritinization and visually inspecting the displayed cells.

43. A method to detect abnormal cells in sample cells of epithelial tissue of the body according to claims 28 - 39, comprising the step of setting a threshold level relating to characteristics of the sample cells and detecting when the characteristics of said sample cells exceed said threshold level.

44. A method to detect abnormal cells in sample cells of epithelial tissue of the body according to claim 43, wherein the characteristics of the sample cells relate to the color characteristics thereof.

45. A method to detect abnormal cells in sample cells of epithelial tissue of the body according to claim 44, wherein the color characteristics relate to color saturation.

46. A method to detect abnormal cells in sample cells of epithelial tissue of the body according to claim 44, wherein the color characteristics relate to the appearance of abnormal keritinization.

47. A method to detect abnormal cells in sample cells of epithelial tissue of the body according to claim 43, wherein the characteristics of the sample cells relate to morphological characteristics.

## Patentansprüche

1. Apparat zum Erfassen anormaler Zellen in Epithelgewebe des Körpers, umfassend:
ein nichtlazerierendes Transepithel-Probenahmemittel zum Abnehmen von Zellen aus mindestens zwei Schichten Epithelgewebe,
einen analytischen Apparat umfassend ein Bilderkennungssystem zum Erfassen und Wiedergeben ausgewählter Zellen der Zellen und zum Analysieren der ausgewählten Zellen, die durch das Transepithel-Probenahmemittel abgenommen worden sind, um anormal keratinisierte Zellen oder Zellen zu erfassen, die eine morphologische Zellveränderung aufweisen, die mit Präkarzinom und Krebs verbunden ist,
wobei das nichtlazerierende Transepithel-Probenahmemittel ausreichend abrasiv ist, um Zellen aus den drei Schichten Epithelgewebe abzunehmen, wobei die drei Schichten die Oberflächen-, Zwischen- und Basalschicht umfassen,
wobei das Bilderkennungssystem geeignet ist, zu erfassen, ob Zellen aus der Basalschicht erfasst werden oder nicht.

2. Apparat zum Erfassen anormaler Zellen im Epithelgewebe des Körpers nach Anspruch 1, wobei das Epithelgewebe keratinisierte Zellen umfasst.

3. Apparat zum Erfassen anormaler Zellen im Epithelgewebe des Körpers nach Anspruch 1 oder 2, wobei der analytische Apparat geeignet ist, die Analyse abzubrechen, wenn keine Zellen aus den Basalschichten erfasst werden.

4. Apparat zum Erfassen anormaler Zellen im Epithelgewebe des Körpers nach einem der vorhergehenden Ansprüche, wobei das Transepithel-Probenahmemittel eine abrasive Probenahmestruktur umfasst.

5. Apparat zum Erfassen anormaler Zellen im Epithelgewebe des Körpers nach Anspruch 4, wobei die Probenahmestruktur einen Pinsel umfasst.

6. Apparat zum Erfassen anormaler Zellen im Epithelgewebe des Körpers nach Anspruch 5, wobei der Pinsel Borsten ausreichender Steifigkeit umfasst, um die Basalschicht zu erreichen.

7. Apparat zum Erfassen anormaler Zellen im Epithelgewebe des Körpers nach Anspruch 6, wobei der Pinsel einen runden steifen Nylonpinsel umfasst.

8. Apparat zum Erfassen von Zellen mit anormaler Morphologie im Epithelgewebe des Körpers nach den Ansprüchen 1 - 3 oder 6, wobei das Epithelgewebe sich in den Bereichen des Körpers ausgewählt aus der Gruppe bestehend aus dem Kehlkopf, dem Rachen, der Speiseröhre und der Vulva befindet.

9. Apparat zum Erfassen von anormalen Zellen im Epithelgewebe des Körpers nach den Ansprüchen 1 - 3, wobei das Bilderkennungssystem geeignet ist, die anormalen Zellen abzubilden.

10. Apparat zum Erfassen von anormalen Zellen im Epithelgewebe des Körpers nach Anspruch 9, wobei das Bilderkennungssystem Mittel, die suspektesten anormalen Zellen unter den als Probe genommenen Zellen auszuwählen und Mittel zum Abbilden der suspekten Zellen umfasst.

11. Apparat zum Erfassen von anormalen Zellen im Epithelgewebe des Körpers nach Anspruch 10, wobei das Bilderkennungssystem des Weiteren Mittel zum Herstellen eines Farbbilds der suspekten Zellen umfasst.

12. Apparat zum Erfassen von anormalen Zellen im Epithelgewebe des Körpers nach den Ansprüchen 9 - 11, wobei das Bilderkennungssystem geeignet ist, die anormalen Zellen, die betrachtet werden sollen, wiederzugeben.

13. Apparat zum Erfassen von anormalen Zellen im Epithelgewebe des Körpers nach den Ansprüchen 9 - 12, wobei das Bilderkennungssystem geeignet ist, Zellen zu erfassen, die durch das Probenahmemittel abgenommen worden sind, die eine anormale Morphologie aufweisen, wobei das Bilderkennungssystem des Weiteren einen algorithmischen Klassierer umfasst.

14. Apparat zum Erfassen von anormalen Zellen im Epithelgewebe des Körpers nach den Ansprüchen 9 - 13, wobei der Apparat des Weiteren Mittel zum Durchführen eines morphologischen Verschlusses umfasst.

15. Apparat zum Erfassen von anormalen Zellen im Epithelgewebe des Körpers nach Anspruch 13, wobei der algorithmische Klassierer eine erste Gruppe von Kandidatenzellen auswählt, wobei der Apparat des weiteren Mittel umfasst, eine Größenbestimmung an der ersten Gruppe von Kandidatenzellen durchzuführen.

16. Apparat zum Erfassen von anormalen Zellen im Epithelgewebe des Körpers nach den Ansprüchen 9 - 15, wobei der Apparat des Weiteren Mittel umfasst, eine zweite Gruppe von Kandidatenzellen herzustellen, die mit den erfassten Farbeigenschaften zusammenhängen.

17. Apparat zum Erfassen von anormalen Zellen im Epithelgewebe des Körpers nach Anspruch 16, von Anspruch 15 abhängend, des Weiteren Mittel zum Analysieren der ersten und zweiten Gruppen von Kandidatenzellen umfassend, um diese Zellen als die höchste Wahrscheinlichkeit aufweisend, anormale Zellen zu sein, einzustufen.

18. Apparat zum Erfassen von anormalen Zellen im Epithelgewebe des Körpers nach Anspruch 17, des Weiteren Mittel umfassend, eine ausgewählte Gruppe der Zellen wiederzugeben, die die höchste Wahrscheinlichkeit aufweisen.

19. Apparat zum Erfassen von anormalen Zellen im Epithelgewebe des Körpers nach den Ansprüchen 15 - 18, wobei der Apparat Mittel umfasst, die Zellgröße von ungefähr 2 - 18 µm im Durchmesser zu erfassen.

20. Apparat zum Erfassen von anormalen Zellen im Epithelgewebe des Körpers nach den Ansprüchen 15 - 18, wobei der Apparat des Weiteren Mittel umfasst, Dinge von weniger als 10 µm im Durchmesser zurückzuweisen.

21. Apparat zum Erfassen von anormalen Zellen im Epithelgewebe des Körpers nach Anspruch 9, wobei der Apparat, der die gefärbten Zellen analysiert, einen algorithmischen Klassierer umfasst, zum Isolieren von Zellkernen von dem durch das Bilderkennungssytem hergestellten Bild, des Weiteren Mittel zum Durchführen eines morphologischen Verschlusses und zum Durchführen einer Größenbestimmung umfassend, um eine erste Gruppe von Kandidatenzellen zu ergeben.

22. Apparat zum Erfassen von anormalen Zellen im Epithelgewebe des Körpers nach Anspruch 9, wobei das Bilderkennungssytem einen algorithmischen Klassierer umfasst, zum Erfassen von Parametern, die mit dem Vorliegen von Keratin verbunden sind, zum Durchführen eines weiteren morphologischen Verschlusses der Probe zum Analysieren des Vorliegens von Keratin und zum Durchführen einer Größenbestimmung, um eine zweite Gruppe von Kandidatenzellen unter den Zellen zu ergeben, die Keratin enthalten und einen morphologischen Verschluss erfahren haben.

23. Apparat zum Erfassen von anormalen Zellen im Epithelgewebe des Körpers nach Anspruch 16, des Weiteren ein neurales Netzwerk zum Analysieren der ersten Gruppe von Kandidatenzellen und der zweiten Gruppe von Kandidatenzellen, um diejenigen, die die höchste anormale Morphologie aufweisen, einzustufen, und Mittel zum Wiedergeben einer Teilmenge der Zellen umfassend.

24. Apparat zum Erfassen von anormalen Zellen im Epithelgewebe des Körpers nach Anspruch 23, wobei das Bildererkennungssystem Mittel umfasst, zum Wiedergeben der am höchsten eingestuften Zellen von der ersten Gruppe von Kandidatenzellen und zum Wiedergeben von Zellen, die das Vorliegen von Farbcharakteristiken aufweisen, die mit einer anormalen Keratinisierung verbunden sind, zur visuellen Untersuchung.

25. Apparat zum Erfassen von anormalen Zellen im Epithelgewebe des Körpers nach Anspruch 23 oder 24, wobei das Bildererkennungssystem und der analytische Apparat Mittel, einen Schwellenwert mit Bezug auf die Charakteristiken der durch das Probenahmemittel als Probe abgenommenen Zellen zu setzen und Mittel zum Erfassen, wann die Charakteristiken der Zellen des Probenahmemittels den Schwellenwert übersteigen, umfassen.

26. Apparat zum Erfassen von anormalen Zellen im Epithelgewebe des Körpers nach Anspruch 25, wobei die Charakteristiken der Zellen, von denen eine Probe genommen worden ist und für die ein Schwellenwert gesetzt worden ist, sich auf die Farbcharakteristiken derselben beziehen.

27. Apparat zum Erfassen von anormalen Zellen im Epithelgewebe des Körpers nach Anspruch 26, wobei die Farbcharakteristiken sich auf das Aussehen anormaler Keritinisation beziehen.

28. Methode zum Erfassen anormaler Zellen in Probezellen des Epithelgewebe des Körpers, wobei derartige Probezellen von nichtlazerierenden Mitteln abgenommen worden sind, die ausreichend abrasiv sind, um die Probezellen unter den Oberflächen-, Zwischen- und Basalschichten des Epithelgewebes abzunehmen, wobei bei der Methode die Probezellen analysiert werden, wobei die Methode Folgendes umfasst:
das Verwenden eines Bilderkennungssystems zum Erfassen und Wiedergeben ausgewählter Zellen der Probezellen und zum Analysieren der ausgewählten Zellen zum Erfassen anormal keratinisierter Zellen oder Zellen, die eine morphologische Zellveränderung aufweisen, die mit Präkarzinom und Krebs verbunden ist, wobei das Verfahren des Weiteren den Schritt des Analysierens der Probezellen zum Bestimmen, ob Probezellen aus der Basalschicht aufgenommen werden sind, umfasst.

29. Methode zum Erfassen anormaler Zellen in Probezellen von Epithelgewebe des Körpers nach Anspruch 28, wobei das Epithelgewebe orale Schleimhaut ist.

30. Methode zum Erfassen anormaler Zellen in Probezellen von Epithelgewebe des Körpers nach entweder Anspruch 28 oder 29, des Weiteren den Schritt des Abbildens der anormalen keratinisierten Zellen umfassend.

31. Methode zum Erfassen anormaler Zellen in Probezellen von Epithelgewebe des Körpers nach den Ansprüchen 28 - 30, des Weiteren den Schritt des Auswählens der suspektesten anormalen Zellen unter den Probezellen umfassend.

32. Methode zum Erfassen anormaler Zellen in Probezellen von Epithelgewebe der Körpers nach den Ansprüchen 28 - 31, des Weiteren den Schritt des Erfassens von Farbeigenschaften, die mit anormaler Keratinisierung verbunden sind, umfassend.

33. Methode zum Erfassen anormaler Zellen in Probezellen von Epithelgewebe des Körpers nach den Ansprüchen 28 - 32, des Weiteren die Schritte des Erfassens, welche der Probezellen eine anormale Morphologie aufweisen, und die Punktebewertung der Zellen, die eine anormale Morphologie aufweisen, umfassend.

34. Methode zum Erfassen anormaler Zellen in Probezellen von Epithelgewebe des Körpers nach Anspruch 32, des Weiteren den Schritt des Durchführens eines morphologischen Verschlusses umfassend.

35. Methode zum Erfassen anormaler Zellen in Probezellen von Epithelgewebe des Körpers nach Anspruch 34, des Weiteren das Durchführen einer Größenbestimmung umfassend, um eine erste Gruppe von Kandidatenzellen zu ergeben.

36. Methode zum Erfassen anormaler Zellen in Probezellen von Epithelgewebe des Körpers nach Anspruch 35, des Weiteren das Herstellen einer zweiten Gruppe von Kandidatenzellen umfassend, die mit Farbsättigungscharakteristiken verbunden sind.

37. Methode zum Erfassen anormaler Zellen in Probezellen von Epithelgewebe des Körpers nach Anspruch 36, des Weiteren den Schritt des Analysierens der ersten und zweiten Gruppen von Kandidatenzellen umfassend, um diejenigen Zellen einzustufen, die die höchste Wahrscheinlichkeit aufweisen, anormale Zellen zu sein.

38. Methode zum Erfassen anormaler Zellen in Probezellen von Epithelgewebe des Körpers nach den Ansprüchen 28 - 37, umfassend den Schritt des Färbens der Zellen, um ein gefärbtes Aussehen von anormalem Keratin zu bilden.

39. Methode zum Erfassen anormaler Zellen in Probezellen von Epithelgewebe des Körpers nach Anspruch 38, umfassend den Schritt des algorithmischen Klassierens der gefärbten Zellen, das Abbilden der gefärbten Zellen, das Isolieren von Zellkernen von dem Bild, das Durchführen eines morphologischen Verschlusses und das Durchführen einer Größenbestimmung, um eine erste Gruppe von Kandidatenzellen zu ergeben.

40. Methode zum Erfassen anormaler Zellen in Probezellen von Epithelgewebe des Körpers nach Anspruch 39, des Weiteren umfassend die Schritte des Durchführens eines morphologischen Verschlusses von Zellen, die mit dem Vorliegen anormaler Keratinisierung verbunden sind, und das Durchführen einer Größenbestimmung der Zellen, die mit dem Vorliegen anormaler Keratinisierung verbunden sind, um eine zweite Gruppe von Kandidatenzellen zu ergeben.

41. Methode zum Erfassen von anormalen Zellen in Probezellen von Epithelgewebe des Körpers nach entweder Anspruch 36 oder Anspruch 37, des Weiteren den Schritt des Analysierens der ersten Gruppe von Kandidatenzellen und der zweiten Gruppe von Kandidatenzellen, um diejenigen, die die höchste anormale Morphologie aufweisen, einzustufen, und das Wiedergeben einer Teilmenge der Zellen umfassend.

42. Methode zum Erfassen von anormalen Zellen in Probezellen von Epithelgewebe des Körpers nach Anspruch 41, des Weiteren umfassend den Schritt des Wiedergebens der am höchsten eingestuften Zellen von der zweiten Gruppe von Kandidatenzellen, die das Vorliegen von Farbcharakteristiken aufweisen, die mit einer anormalen Keratinisierung verbunden sind und das visuelle Untersuchung der wiedergegebenen Zellen.

43. Methode zum Erfassen von anormalen Zellen in Probezellen von Epithelgewebe des Körpers nach Anspruch 28 - 39, umfassend den Schritt des Einstellens eines Schwellenwerts mit Bezug auf die Charakteristiken der Probezellen und das Erfassen, wann die Charakteristiken der Probezellen den Schwellenwert übersteigen.

44. Methode zum Erfassen von anormalen Zellen in Probezellen von Epithelgewebe des Körpers nach Anspruch 43, wobei die Charakteristiken der Probenzellen sich auf die Farbcharakteristiken davon beziehen.

45. Methode zum Erfassen anormaler Zellen in Probezellen von Epithelgewebe des Körpers nach Anspruch 44, wobei die Farbcharakteristiken sich auf die Farbsättigung beziehen.

46. Methode zum Erfassen anormaler Zellen in Probezellen von Epithelgewebe des Körpers nach Anspruch 44, wobei die Farbcharakteristiken sich auf das Aussehen anormaler Keratinisierung beziehen.

47. Methode zum Erfassen anormaler Zellen in Probezellen von Epithelgewebe des Körpers nach Anspruch 43, wobei die Charakteristiken der Probezellen sich auf die morphologischen Charakteristiken beziehen.

## Revendications

1. Appareil servant à détecter des cellules anormales dans du tissu épithélial de l'organisme, comprenant :
un moyen d'échantillonnage transépithélial sans lacération servant à prélever des cellules dans au moins deux couches dudit tissu épithélial,
un appareil analytique comprenant un système de reconnaissance d'images pour détecter et visualiser les cellules sélectionnées desdites cellules et pour analyser lesdites cellules sélectionnées prélevées par ledit moyen d'échantillonnage transépithélial afin de détecter les cellules anormalement kératinisées, ou les cellules présentant un changement cellulaire morphologique associé au précancer et au cancer,
dans lequel ledit moyen d'échantillonnage transépithélial sans lacération est suffisamment abrasif pour prélever des cellules des trois couches dudit tissu épithélial, lesdites trois couches comprenant les couches superficielle, intermédiaire et basale,
dans lequel le système de reconnaissance d'images est adapté de manière à détecter si des cellules de ladite couche basale sont détectées ou non.

2. Appareil servant à détecter des cellules anormales dans le tissu épithélial de l'organisme selon la revendication 1, dans lequel ledit tissu épithélial comprend des cellules kératinisées.

3. Appareil servant à détecter des cellules anormales dans le tissu épithélial de l'organisme selon la revendication 1 ou 2, dans lequel ledit appareil analytique est adapté de manière à mettre fin à ladite analyse si des cellules desdites couches basales ne sont pas détectées.

4. Appareil servant à détecter des cellules anormales dans le tissu épithélial de l'organisme selon l'une quelconque des revendications précédentes, dans lequel ledit moyen d'échantillonnage transépithélial comprend une structure d'échantillonnage abrasive.

5. Appareil servant à détecter des cellules anormales dans le tissu épithélial de l'organisme selon la revendication 4, dans lequel ladite structure d'échantillonnage comprend une brosse.

6. Appareil servant à détecter des cellules anormales dans le tissu épithélial de l'organisme selon la revendication 5, dans lequel ladite brosse comprend des soies d'une raideur suffisante pour atteindre ladite couche basale.

7. Appareil servant à détecter des cellules anormales dans le tissu épithélial de l'organisme selon la revendication 6, dans lequel ladite brosse comprend une brosse en nylon circulaire dure.

8. Appareil servant à détecter des cellules présentant une morphologie anormale dans le tissu épithélial de l'organisme selon les revendications 1 à 3 ou 6, dans lequel ledit tissu épithélial se situe dans les zones de l'organisme sélectionnées parmi le groupe constitué par le larynx, le pharynx, l'oesophage et la vulve.

9. Appareil servant à détecter des cellules anormales dans le tissu épithélial de l'organisme selon les revendications 1 à 3, le système de reconnaissance d'images étant adapté de manière à visualiser lesdites cellules anormales.

10. Appareil servant à détecter des cellules anormales dans le tissu épithélial de l'organisme selon la revendication 9, dans lequel ledit système de reconnaissance d'images comprend un moyen pour sélectionner les cellules anormales les plus suspectes parmi les cellules prélevées et un moyen de visualiser lesdites cellules suspectes.

11. Appareil servant à détecter des cellules anormales dans le tissu épithélial de l'organisme selon la revendication 10, dans lequel ledit système de reconnaissance d'images comprend, en outre, un moyen pour produire une image en couleur desdites cellules suspectes.

12. Appareil servant à détecter des cellules anormales dans le tissu épithélial de l'organisme selon les revendications 9 à 11, dans lequel ledit système de reconnaissance d'images est adapté de manière à visualiser lesdites cellules anormales à examiner.

13. Appareil servant à détecter des cellules anormales dans le tissu épithélial de l'organisme selon les revendications 9 à 12, dans lequel ledit système de reconnaissance d'images est adapté de manière à détecter les cellules prélevées par ledit moyen d'échantillonnage qui présentent une morphologie anormale, ledit système de reconnaissance d'images comprenant, en outre, un classificateur algorithmique.

14. Appareil servant à détecter des cellules anormales dans le tissu épithélial de l'organisme selon les revendications 9 à 13, dans lequel ledit appareil comprend, en outre, un moyen pour réaliser une fermeture morphologique.

15. Appareil servant à détecter des cellules anormales dans le tissu épithélial de l'organisme selon la revendication 13, dans lequel ledit classificateur algorithmique sélectionne un premier groupe de cellules candidates, ledit appareil comprenant, en outre, un moyen pour calibrer ledit premier groupe de cellules candidates.

16. Appareil servant à détecter des cellules anormales dans le tissu épithélial de l'organisme selon les revendications 9 à 15, dans lequel ledit appareil comprend, en outre, un moyen pour produire un second groupe de cellules candidates associé aux propriétés des couleurs détectées.

17. Appareil servant à détecter des cellules anormales dans le tissu épithélial de l'organisme selon la revendication 16, telle que dépendant de la revendication 15, comprenant, en outre, un moyen pour analyser lesdits premier et second groupes de cellules candidates en vue de classer les cellules ayant la plus forte probabilité d'être des cellules anormales.

18. Appareil servant à détecter des cellules anormales dans le tissu épithélial de l'organisme selon la revendication 17, comprenant, en outre, un moyen de visualiser un groupe sélectionné desdites cellules ayant ladite plus forte probabilité.

19. Appareil servant à détecter des cellules anormales dans le tissu épithélial de l'organisme selon les revendications 15 à 18, dans lequel ledit appareil comprend un moyen pour détecter une taille de cellule d'un diamètre approximatif de 2 à 18 µm.

20. Appareil servant à détecter des cellules anormales dans le tissu épithélial de l'organisme selon les revendications 15 à 18, dans lequel ledit appareil comprend un moyen pour rejeter les objets d'un diamètre inférieur à 10 µm.

21. Appareil servant à détecter des cellules anormales dans le tissu épithélial de l'organisme selon la revendication 9, dans lequel ledit appareil qui analyse lesdites cellules colorées inclut un classificateur algorithmique pour isoler les noyaux de cellules à partir de l'image produite par ledit système de reconnaissance d'images comprenant, en outre, un moyen pour réaliser une fermeture morphologique et pour effectuer un calibrage pour obtenir un premier groupe de cellules candidates.

22. Appareil servant à détecter des cellules anormales dans le tissu épithélial de l'organisme selon la revendication 9, dans lequel ledit système de reconnaissance d'images inclut un classificateur algorithmique pour détecter les paramètres associés à la présence de kératine, pour réaliser une autre fermeture morphologique dudit échantillon analysant la présence de kératine, et pour effectuer un calibrage afin d'obtenir un second groupe de cellules candidates à partir desdites cellules qui contiennent de la kératine et qui ont eu une fermeture morphologique.

23. Appareil servant à détecter des cellules anormales dans le tissu épithélial de l'organisme selon la revendication 16, comprenant, en outre, un réseau neural pour analyser ledit premier groupe de cellules candidates et ledit second groupe de cellules candidates pour classer celles présentant la morphologie la plus anormale, et un moyen pour visualiser un sous-groupe desdites cellules.

24. Appareil servant à détecter des cellules anormales dans le tissu épithélial de l'organisme selon la revendication 23, dans lequel ledit système de reconnaissance d'images comprend un moyen pour visualiser les cellules de la classe supérieure appartenant au premier groupe de cellules candidates et visualiser les cellules montrant la présence de caractéristiques de couleurs associées à la kératinisation anormale pour l'inspection visuelle.

25. Appareil servant à détecter des cellules anormales dans le tissu épithélial de l'organisme selon la revendication 23 ou 24, dans lequel ledit système de reconnaissance d'images et ledit appareil analytique comprend un moyen pour fixer un niveau seuil en rapport avec les caractéristiques des cellules prélevées par ledit moyen d'échantillonnage, et un moyen pour détecter quand les caractéristiques desdites cellules dudit moyen d'échantillonnage dépassent ledit niveau seuil.

26. Appareil servant à détecter des cellules anormales dans le tissu épithélial de l'organisme selon la revendication 25, dans lequel les caractéristiques des cellules prélevées pour lesquelles il est fixé un niveau seuil se rapportent aux caractéristiques des couleurs de ces dernières.

27. Appareil servant à détecter des cellules anormales dans le tissu épithélial de l'organisme selon la revendication 26, dans lequel les caractéristiques des couleurs se rapportent à l'aspect de la kératinisation anormale.

28. Méthode servant à détecter des cellules anormales dans les cellules échantillons de tissu épithélial de l'organisme, de telles cellules échantillons ayant été prélevées par des moyens sans lacération suffisamment abrasifs pour prélever lesdites cellules échantillons parmi les couches superficielle, intermédiaire et basale dudit tissu épithélial, dans laquelle ladite méthode analyse les cellules échantillons, ladite méthode comprenant les étapes qui consistent à :
utiliser un système de reconnaissance d'images pour détecter et visualiser les cellules sélectionnées desdites cellules échantillons et pour analyser les cellules sélectionnées pour détecter les cellules anormalement kératinisées ou les cellules présentant un changement cellulaire morphologique associé au précancer et au cancer, dans laquelle ladite méthode inclut, en outre, l'étape qui consiste à analyser les cellules échantillons afin de déterminer si les cellules échantillons ont été prélevées dans la couche basale.

29. Méthode servant à détecter des cellules anormales dans les cellules échantillons de tissu épithélial de l'organisme selon la revendication 28, dans laquelle ledit tissu épithélial est la muqueuse buccale.

30. Méthode servant à détecter des cellules anormales dans les cellules échantillons de tissu épithélial de l'organisme selon la revendication 28 ou la revendication 29, comprenant, en outre, l'étape qui consiste à visualiser lesdites cellules anormalement kératinisées.

31. Méthode servant à détecter des cellules anormales dans les cellules échantillons de tissu épithélial de l'organisme selon les revendications 28 à 30, comprenant, en outre, l'étape qui consiste à sélectionner les cellules anormales les plus suspectes parmi les cellules échantillons.

32. Méthode servant à détecter des cellules anormales dans les cellules échantillons de tissu épithélial de l'organisme selon les revendications 28 à 31, comprenant, en outre, l'étape qui consiste à détecter les propriétés des couleurs associées à la kératinisation anormale.

33. Méthode servant à détecter des cellules anormales dans les cellules échantillons de tissu épithélial de l'organisme selon les revendications 28 à 32, comprenant, en outre, les étapes qui consistent à détecter, parmi lesdites cellules échantillons, celles qui présentent une morphologie anormale, et à coter lesdites cellules qui présentent une morphologie anormale.

34. Méthode servant à détecter des cellules anormales dans les cellules échantillons de tissu épithélial de l'organisme selon la revendication 32, comprenant, en outre, l'étape qui consiste à effectuer une fermeture morphologique.

35. Méthode servant à détecter des cellules anormales dans les cellules échantillons de tissu épithélial de l'organisme selon la revendication 34, comprenant, en outre, la réalisation d'un calibrage afin d'obtenir un premier groupe de cellules candidates.

36. Méthode servant à détecter des cellules anormales dans les cellules échantillons de tissu épithélial de l'organisme selon la revendication 35, comprenant, en outre, la production d'un second groupe de cellules candidates associées aux caractéristiques de saturation des couleurs.

37. Méthode servant à détecter des cellules anormales dans les cellules échantillons de tissu épithélial de l'organisme selon la revendication 36, comprenant, en outre, l'étape qui consiste à analyser lesdits premier et second groupes de cellules candidates pour classer ces cellules comme ayant la plus forte probabilité d'être des cellules anormales.

38. Méthode servant à détecter des cellules anormales dans les cellules échantillons de tissu épithélial de l'organisme selon les revendications 28 à 37, comprenant l'étape de coloration desdites cellules pour produire un aspect coloré de la kératine anormale.

39. Méthode servant à détecter des cellules anormales dans les cellules échantillons de tissu épithélial de l'organisme selon la revendication 38, comprenant l'étape de classification algorithmique desdites cellules colorées, de visualisation desdites cellules colorées, d'isolement des noyaux des cellules à partir de ladite visualisation, de réalisation d'une fermeture morphologique et de réalisation d'un calibrage afin d'obtenir un premier groupe de cellules candidates.

40. Méthode servant à détecter des cellules anormales dans les cellules échantillons de tissu épithélial de l'organisme selon la revendication 39, comprenant, en outre, les étapes qui consistent à effectuer une fermeture morphologique des cellules associées à la présence d'une kératinisation anormale et à effectuer un calibrage desdites cellules associées à la présence d'une kératinisation anormale afin d'obtenir un second groupe de cellules candidates.

41. Méthode servant à détecter des cellules anormales dans les cellules échantillons de tissu épithélial de l'organisme selon la revendication 36 ou la revendication 37, comprenant, en outre, l'étape qui consiste à analyser ledit premier groupe de cellules candidates et ledit second groupe de cellules candidates afin de classer celles présentant la morphologie la plus anormale et de visualiser un sous-groupe desdites cellules.

42. Méthode servant à détecter des cellules anormales dans les cellules échantillons de tissu épithélial de l'organisme selon la revendication 41, comprenant, en outre, l'étape qui consiste à visualiser les cellules de la classe supérieure appartenant au second groupe de cellules candidates qui présentent les caractéristiques des couleurs associées à la kératinisation anormale et à inspecter visuellement les cellules visualisées.

43. Méthode servant à détecter des cellules anormales dans les cellules échantillons de tissu épithélial de l'organisme selon les revendications 28 à 39, comprenant l'étape qui consiste à fixer un niveau seuil associé aux caractéristiques des cellules échantillons et à détecter quand les caractéristiques desdites cellules échantillons dépassent ledit niveau seuil.

44. Méthode servant à détecter des cellules anormales dans les cellules échantillons de tissu épithélial de l'organisme selon la revendication 43, dans laquelle les caractéristiques des cellules échantillons ont trait aux caractéristiques des couleurs de celles-ci.

45. Méthode servant à détecter des cellules anormales dans les cellules échantillons de tissu épithélial de l'organisme selon la revendication 44, dans laquelle les caractéristiques des couleurs ont trait à la saturation des couleurs.

46. Méthode servant à détecter des cellules anormales dans les cellules échantillons de tissu épithélial de l'organisme selon la revendication 44, dans laquelle les caractéristiques des couleurs ont trait à l'aspect de la kératinisation anormale.

47. Méthode servant à détecter des cellules anormales dans les cellules échantillons de tissu épithélial de l'organisme selon la revendication 43, dans laquelle les caractéristiques des cellules échantillons ont trait aux caractéristiques morphologiques.
